# EUROPÄISCHE PATENTSCHRIFT

(11) **EP 2 621 661 B1**
(45) Veröffentlichungstag und Bekanntmachung des Hinweises auf die Patenterteilung: **01.03.2023**
(21) Anmeldenummer: 10763130.1
(22) Anmeldetag: 30.09.2010
(51) Int. Cl.: B23K 26/402

(54) **VERFAHREN ZUM PRÜFEN EINER LASEREINRICHTUNG**
METHOD FOR TESTING A LASER DEVICE
PROCÉDÉ DE TEST D'UN DISPOSITIF LASER

(43) Veröffentlichungstag der Anmeldung: 07.08.2013
(73) Patentinhaber: Alcon Inc., 1701 Fribourg (CH)
(72) Erfinder: GORSCHBOTH, Claudia, 90411 Nuernberg (DE); WÖLFEL, Mathias, 91052 Erlangen (DE); HEIMISCH, Richard, 90763 Fuerth (DE); DONITZKY, Christof, 90542 Eckental/Eschenau (DE); KITTELMANN, Olaf, 14153 Berlin (DE); DEISINGER, Thomas, 90556 Cadolzburg (DE); ROBL, Gerhard, 90547 Stein (DE); VOGLER, Klaus, 90542 Eckental/Eschenau (DE); STARIGK, Martin, 90478 Nuernberg (DE)
(74) Vertreter: Katérle, Axel
(86) Internationale Anmeldenummer: PCT/EP2010/005971
(87) Internationale Veröffentlichungsnummer: WO 2012/041346

(56) Entgegenhaltungen:
- WO-A1-2008/040436
- WO-A2-2009/059251
- DE-A1-102007 056 554
- US-A1- 2004 070 761
- US-A1- 2010 228 236

## Beschreibung

Die Erfindung befasst sich mit Methoden zum Prüfen einer zur Objektbearbeitung nutzbaren Lasereinrichtung, welche zur Abgabe gepulster fokussierter Laserstrahlung eingerichtet ist. Insbesondere sollen die Methoden eine Überprüfung der Positioniergenauigkeit des Strahlungsfokus der Laserstrahlung ermöglichen.

Zur Bearbeitung von Objekten in der Tiefe des Objektmaterials ist es bekannt, ultrakurzpulsige Laserstrahlung mit Pulsdauern im Femtosekundenbereich (gegebenenfalls bis in den einstelligen Pikosekundenbereich) zu verwenden, die im Fokus einen laserinduzierten optischen Durchbruch und daraus resultierend eine im wesentlichen auf das Fokusgebiet beschränkte Photodisruption bewirken kann. Voraussetzung hierfür ist eine Transparenz des bearbeiteten Objekts für die Laserstrahlung, was beispielsweise bei Bearbeitungen des humanen Auges oberhalb einer Wellenlänge von etwa 300 nm gegeben ist. Bei der Laserbearbeitung des menschlichen Auges wird Fs-Laserstrahlung insbesondere zur Erzeugung von Schnitten in der Kornea oder anderen Gewebeteilen des Auges eingesetzt, etwa im Rahmen einer LASIK-Behandlung (LASIK: Laser In-Situ Keratomileusis) zur Erzeugung eines Flaps, bei einer kornealen Lentikelextraktion zur Erzeugung einer linsenförmigen intrakornealen Lamelle oder bei einer kornealen Keratoplastik zum Ausschneiden eines zu ersetzenden oder zu verpflanzenden kornealen Gewebestücks.

Bei all diesen Bearbeitungsformen ist eine hohe Positioniergenauigkeit des Laserfokus in allen drei Raumkoordinaten im Zielgewebe erforderlich, wobei derzeit übliche Genauigkeitsanforderungen einige wenige µm vorsehen und im bestmöglichen Fall Positioniertoleranzen von nur 1 oder 2 µm zulassen.

Zumindest die in der Laserchirurgie des humanen Auges eingesetzten Fs-Lasereinrichtungen besitzen oftmals eine gelegentlich als Patientenadapter bezeichnete mechanische Schnittstelleneinheit mit einem für die Laserstrahlung transparenten Kontaktelement, das eine Kontaktfläche aufweist, welche in flächigen Anlagekontakt mit der Augenoberfläche oder allgemein mit dem zu bearbeitenden Objekt zu bringen ist. Die Schnittstelleneinheit ist beispielsweise ein auswechselbares Modul, das an eine Fokussieroptik der Lasereinrichtung angekoppelt werden kann. Das Kontaktelement mit seiner Kontaktfläche kann als Positionsreferenz für die Einstellung der Position des Strahlungsfokus dienen. Indem das Auge an das Kontaktlement angelegt wird, ist - eine genaue Referenzierung der Fokusposition gegenüber der Kontaktfläche vorausgesetzt - eine präzise Bearbeitung des Auges möglich.

Die Erfindung zielt darauf ab, dem Anwender einer Fs-Lasereinrichtung einen Routinetest zur Verfügung zu stellen, der eine einfache Überprüfung der Genauigkeit der Fokuspositionierung, insbesondere in Ausbreitungsrichtung der Laserstrahlung (nachfolgend z-Richtung genannt), ermöglicht. Vorzugsweise sollen der Test und sein Ergebnis in einfacher Weise dokumentierbar sein.

US 2006/0114469 A1 schlägt vor, zur Überprüfung der räumlichen Lage und Orientierung der Kontaktfläche eines als Applanationsplatte ausgebildeten Kontaktelements einer Lasereinrichtung den Strahlungsfokus längs vorgegebener Kreisbahnen zu bewegen und Plasmafunken, die auftreten, wenn der Fokus am Rand der Applanationsplatte auf diese trifft, mit einem Photodetektor zu registrieren.

WO 2009/059251 A2, die die Grundlage für den Oberbegriff des Anspruchs 1 darstellt, lehrt in einem Ausführungsbeispiel ein Verfahren zur präzisen räumlichen Justierung eines Femtosekunden-Lasers und eines OCT-basierten Bildgebungsverfahrenssystems für den Einsatz bei Augenoperationen. Die Justierung erfolgt mittels eines vorkalibrierten, zylinderförmigen Targets oder Phantoms, welches eine 3D Gitterstruktur aufweist, mit deren Hilfe die Position des Laserspots und die Position des OCT-Koordinatensystems bestimmt werden. Um die Justierung durchzuführen werden im Phantom mit Hilfe des Lasers zunächst Markierungsstrukturen in Form eines 3D-Gitters erzeugt und diese anschließend mit dem OCT-Koordinatensystem verglichen.

Zum weiteren Stand der Technik wird auf die US 2004/070761 A1 und die WO 2008/040436 A1 verwiesen.

Die Erfindung stellt demgegenüber ein Verfahren nach Anspruch 1 zum Prüfen der Genauigkeit der Fokuspositionierung einer zur Abgabe gepulster fokussierter Laserstrahlung eingerichteten Lasereinrichtung bereit, deren Fokusposition sowohl in als auch quer zur Ausbreitungsrichtung der Laserstrahlung verstellbar ist, wobei die Lasereinrichtung ein für die Laserstrahlung transparentes Kontaktelement mit einer Anlagefläche zur Anlage eines zu bearbeitenden Objekts aufweist, wobei das Verfahren die Schritte umfasst:
- Anlegen eines zumindest in einem Bearbeitungsbereich für die Laserstrahlung transparenten Testobjekts an die Anlagefläche,
- Einstrahlen der Laserstrahlung in das an der Anlagefläche anliegende Testobjekt und dabei Bewegen der Fokusposition nach Maßgabe eines vorgegebenen Testmusters zur Erzeugung dauerhafter Bearbeitungsstrukturen in dem Testobjekt, wobei die Bearbeitungsstrukturen mindestens eine zu umliegenden Materialbereichen des Testobjekts optisch kontrastierende erste Verfärbungsstruktur umfassen, welche in dem Testobjekt längs einer quer, insbesondere geradlinig schräg, zur Ausbreitungsrichtung der Laserstrahlung verlaufenden Strukturerstreckungsrichtung bis zu einer der Anlagefläche zugekehrten Außenfläche des Testobjekts aufsteigt, wobei die Bearbeitungsstrukturen ferner eine zu umliegenden Materialbereichen des Testobjekts optisch kontrastierende zweite Verfärbungsstruktur umfassen, welche eine oder mehrere Referenzmarkierungen für einen Soll-Durchstoßbereich der ersten Verfärbungsstruktur durch die Außenfläche des Testobjekts bildet, und wobei geprüft wird, ob die erste Verfärbungsstruktur die Außenfläche des Testobjekts innerhalb oder außerhalb eines Durchstoßbereiches durchstößt.

Die Erfindung gibt dem Nutzer eine einfach anwendbare Methode an die Hand, mit der in der täglichen Routine festgestellt werden kann, ob die Lasereinrichtung die gestellten Anforderungen an die Positioniergenauigkeit des Strahlungsfokus einhält. Für diesen Test wird ein geeignetes Testobjekt (Referenzprobe) bereitgestellt, das nach Durchführung des Tests aufbewahrt und langfristig archiviert werden kann. Weil im Rahmen des Tests dauerhafte Bearbeitungsstrukturen in dem Testobjekt erzeugt werden, lassen sich der Test und sein Ergebnis auch zu einem späteren Zeitpunkt ohne weiteres nachvollziehen. Das Testobjekt kann beispielsweise scheiben- oder plattenförmig sein. Als für die Laserstrahlung transparentes Material des Testobjekts eignet sich beispielsweise PMMA (Polymethylmethacrylat), wenngleich anderen Materialien, insbesondere andere transparente, nicht absorbierende Kunststoffmaterialien, keineswegs ausgeschlossen sind.

Wenn hier von einer Verfärbung die Rede ist, so ist dies nicht als Hinweis auf das Entstehen oder Verändern einer echten Farbe zu verstehen. Nachdem die eingestrahlte Strahlungsenergie zu einer lokalen Photodisruption des Materials des Testobjekts führen kann, kann die Verfärbung beispielsweise in einer bloßen lokalen Verdunkelung (Schwärzung) oder dem Entstehen einer milchig-trüben Stelle bestehen. Die Verfärbung kann demnach in einer lokalen Helligkeitsänderung oder einer Graustufenänderung des Materials des Testobjekts durch eine beliebige von der Laserstrahlung ausgelöste Materialmodifikation bestehen. Jedenfalls bewirkt bei dieser Ausgestaltung die Wechselwirkung der Laserstrahlung mit dem Material des Testobjekts das Entstehen einer mit dem bloßen Auge oder/und einem kamerabasierten Bilderkennungssystem detektierbaren Zone, die sich optisch gegenüber den umliegenden Materialbereichen des Testobjekts abhebt und dementsprechend einen optischen Kontrast zu diesen umliegenden Materialbereichen bildet.

Die erste Verfärbungsstruktur kann beispielsweise ein Streifenmuster aus einer Vielzahl längs der Strukturerstreckungsrichtung aufeinanderfolgender Verfärbungsstreifen repräsentieren. Alternativ kann die erste Verfärbungsstruktur eine schräg zur Ausbreitungsrichtung der Laserstrahlung bis zur Außenfläche des Testobjekts aufsteigende ebene Verfärbungsfläche repräsentieren.

Im Fall der Ausgestaltung der ersten Verfärbungsstruktur als Streifenmuster sind die Verfärbungsstreifen mit ihrer Streifenebene vorzugsweise orthogonal zur Strahlungsausbreitungsrichtung orientiert. Paarweise aufeinanderfolgende Streifen können dabei einen gegenseitigen Abstand in Strahlungsausbreitungsrichtung von höchstens 10 µm, besser höchstens 8 µm, noch besser höchstens 6 µm und beispielsweise 5 µm haben. Bei Betrachtung einer Projektion des Streifenmusters auf eine zur Strahlungsausbreitungsrichtung orthogonale Ebene können paarweise aufeinanderfolgende Streifen gegenseitigen Abstand haben. Es ist freilich nicht ausgeschlossen, dass bei einer derartigen Projektionsbetrachtung paarweise aufeinanderfolgende Streifen im wesentlichen abstandsfrei voneinander sind.

Abhängig von der Lage des Durchstoßbereichs der ersten Verfärbungsstruktur relativ zu den Referenzmarkierungen ist eine Aussage über die Qualität der z-Kalibrierung der Lasereinrichtung möglich. Vorteilhafterweise markieren die Referenzmarkierungen einen Soll-Durchstoßbereich der ersten Verfärbungsstruktur durch die Außenfläche des Testobjekts. Je nachdem, ob die erste Verfärbungsstruktur innerhalb oder außerhalb des Soll-Durchstoßbereichs die Außenfläche des Testobjekts durchstößt, kann der Test als bestanden bzw. nicht bestanden qualifiziert werden. Eine solche Auswertung ist sowohl für den Nutzer als auch ein automatisiertes, kamerabasiertes Auswertesystem besonders einfach. Zur Markierung des Soll-Durchstoßbereichs können die Referenzmarkierungen beispielsweise ein paar parallel im Abstand nebeneinander verlaufender Markierungslinien bilden.

Das Testmuster kann die Erzeugung einer zu umliegenden Materialbereichen des Testobjekts optisch kontrastierenden dritten Verfärbungsstruktur vorsehen, welche an der Außengrenze eines vorgegebenen verfügbaren, zur Strahlungsausbreitungsrichtung orthogonalen Positionierfelds für die Fokusposition verläuft. Dieses verfügbare Positionierfeld repräsentiert den maximalen Scanbereich, in dem der Strahlungsfokus nominell in einer Querebene zur Strahlungsausbreitungsrichtung (nachfolgend x,y-Ebene genannt) verstellt werden kann. Die Grenzen dieses Scanbereichs können beispielsweise durch konstruktive oder sonstige strukturelle Gegebenheiten des zur x,y-Ablenkung der Laserstrahlung verwendeten Scanners definiert sein oder/und durch steuerungstechnische Vorgaben. Häufig ist der maximale Scanbereich in der x,y-Ebene durch einen Kreis definiert. Indem die dritte Verfärbungsstruktur unmittelbar an der Außengrenze des nominell verfügbaren maximalen x,y-Scanbereichs erzeugt wird, ist leicht erkennbar, ob der maximale Scanbereich tatsächlich von dem Scanner angefahren werden kann. Treten nämlich Unterbrechungen in der dritten Verfärbungsstruktur auf, ist dies ein Hinweis darauf, dass in dem Unterbrechungsbereich der nominell verfügbare maximale Scanbereich tatsächlich nicht voll ausnutzbar ist.

Vorzugsweise ist das Testobjekt zumindest in seinem Bearbeitungsbereich aus einem im sichtbaren Wellenlängenbereich und bei der Wellenlänge der Laserstrahlung transparenten Material gefertigt.

Das Testobjekt kann zumindest in seinem Bearbeitungsbereich homogen ausgebildet sein. Alternativ ist vorstellbar, dass das Testobjekt zumindest in seinem Bearbeitungsbereich mehrschichtig aufgebaut ist und die Wechselwirkungsreaktion auf die eingestrahlte Laserstrahlung in unterschiedlichen Materialschichten des Testobjekts unterschiedlich ist.

Das Verfahren kann ferner ein Freigeben der Lasereinrichtung für eine Laserbehandlung des humanen Auges umfassen, falls ein Erfolg der Prüfung der Lasereinrichtung festgestellt wird, oder ein Sperren der Lasereinrichtung für eine Laserbehandlung des humanen Auges, falls ein Misserfolg der Prüfung der Lasereinrichtung festgestellt wird. Dieses Freigeben oder Sperren kann durch eine programmgesteuerte Steuereinheit der Lasereinrichtung bewirkt werden, wobei eine Sperrung der Lasereinrichtung beispielsweise nur durch eine nachfolgende erfolgreiche Durchführung eines erneuten Tests aufgehoben werden kann. Die Feststellung, ob der Test erfolgreich durchgeführt wurde oder nicht, kann die Steuereinheit beispielsweise auf Grundlage einer Benutzereingabe über ein Eingabegerät der Lasereinrichtung treffen. Hierzu kann die Steuereinheit beispielsweise den Anwender mittels einer entsprechenden Aufforderung auf einem Monitor dazu auffordern, seine eigene Beurteilung des Testergebnisses einzugeben, etwa über eine Tastatur, ein Zeigegerät oder eine sonstige Form von Eingabegerät. Je nachdem, welche Bewertung der Anwender eingibt, stellt die Steuereinheit fest, ob die Prüfung erfolgreich war oder nicht, und bewirkt daraufhin eine Freigabe oder Sperrung der Lasereinrichtung. Im Zuge der Archivierung des Testobjekts kann auch die vom Anwender eingegebene Bewertung abgespeichert werden.

Die Erfindung wird nachfolgend anhand der beigefügten Zeichnungen weiter erläutert. Es stellen dar:
Figur 1 ein stark schematisiertes Blockdiagram einer Laserbearbeitungseinrichtung bei Durchführung eines Kalibrierungstests gemäß einem Ausführungsbeispiel,
Figur 2 schematisch ein für den Kalibrierungstest verwendetes Testobjekt mit darin eingebrachten Bearbeitungsstrukturen gemäß einem Ausführungsbeispiel,
Figur 3 Details der in das Testobjekt der Figur 2 eingebrachten Bearbeitungsstrukturen,
Figuren 4a bis 4c schematisch unterschiedliche Testergebnisse,
Figur 5 ein Beispiel für Referenzmarkierungen, die im Rahmen eines Kalibrierungstests als Teil der Bearbeitungsstrukturen in ein Testobjekt eingebracht werden können,
Figur 6 ein Testobjekt mit einer darin eingebrachten Bearbeitungsstruktur gemäß einem weiteren Ausführungsbeispiel,
Figuren 7a und 7b beispielhafte Testergebnisse für ein mit den Referenzmarkierungen der Figur 5 versehenes Testobjekt,
Figur 8 ein Testobjekt gemäß einem weiteren Ausführungsbeispiel,
Figur 9 schematisch ein Beispiel für eine Zerteilung eines Testobjekts in Teilobjekte im Rahmen eines Kalibrierungstests, der kein Teil der Erfindung darstellt, und
Figur 10 ein weiteres Beispiel für ein zerteiltes Testobjekt, das kein Teil der Erfindung darstellt,

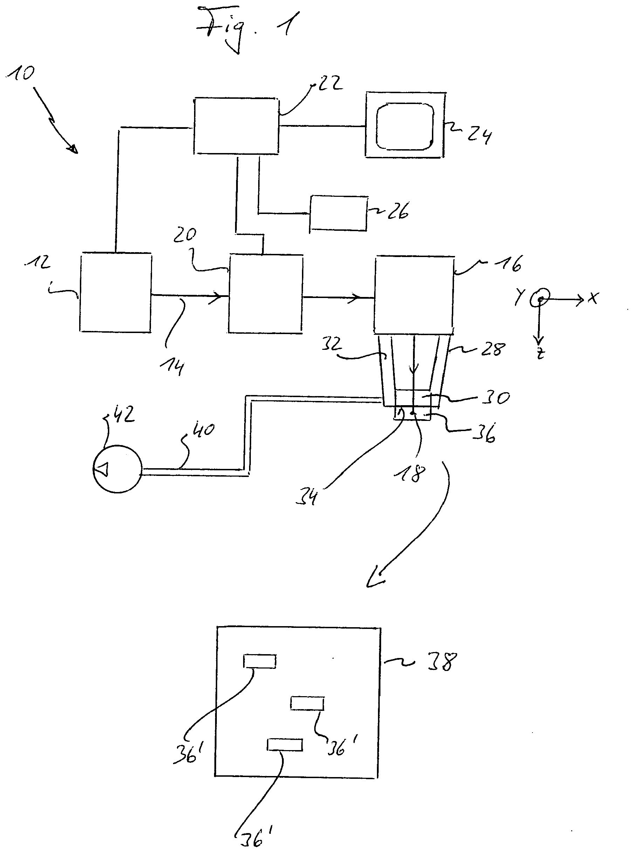

Es wird zunächst auf Figur 1 verwiesen. Die dort gezeigte Laserbearbeitungseinrichtung - allgemein mit 10 bezeichnet - dient zur lasertechnischen Anbringung von Schnitten im menschlichen Auge. Es versteht sich, dass dies nur ein beispielhafter Anwendungsfall ist; grundsätzlich kann die Laserbearbeitungseinrichtung 10 auch für andere Bearbeitungszwecke dienen.

Die Laserbearbeitungseinrichtung 10 umfasst eine Laserquelle 12, welche einen gepulsten Laserstrahl 14 mit Pulsdauern im Femtosekundenbereich, etwa im dreistelligen Femtosekundenbereich, abgibt. Eine Fokussieroptik 16 fokussiert den Laserstrahl 14 auf einen Fokuspunkt 18, wobei die Position des Strahlfokus 18 in Strahlausbreitungsrichtung (z-Richtung) sowie in einer Ebene senkrecht hierzu (x,y-Ebene) mittels Scankomponenten 20 verstellbar ist, welche hier zum Zwecke der besseren Übersichtlichkeit als einheitlicher Funktionsblock dargestellt sind. Die Laserquelle 12 und die Scankomponenten 20 sind durch eine programmgesteuerte Steuereinheit 22 steuerbar, an welche ein Monitor 24 sowie ein Eingabegerät 26 (z.B. Tastatur, Zeigevorrichtung, etc.) angeschlossen sind.

Zur positionsgenauen Einkopplung der Laserstrahlung in das zu bearbeitende Auge besitzt die Laserbearbeitungseinrichtung 10 eine lösbar mit einem Gehäuse der Fokussieroptik 16 gekoppelte Schnittstelleneinheit (Patientenadapter) 28, welche ein für die Laserstrahlung transparentes Kontaktelement 30 sowie einen Halter 32 für das Kontaktelement 30 aufweist. Im gezeigten Beispielfall ist das Kontaktelement 30 als planparallele Applanationsplatte ausgeführt, es kann jedoch auf seiner augenzugewandten Seite oder/und auf seiner augenabgewandten Seite anders gestaltet sein, z.B. konkav oder konvex. Die augenzugewandte Seite des Kontaktelements 30 bildet eine Anlagefläche 34, welche als Positionsreferenz für das zu behandelnde Auge dienen kann. Zur Behandlung eines Auges wird dieses in Anlagekontakt mit der Anlagefläche 34 gebracht, wobei in an sich bekannter Weise zuvor auf das Auge ein nicht näher dargestellter Saugring aufgesetzt werden kann, der seinerseits beispielsweise durch Saugkraft fest mit der Schnittstelleneinheit 28 koppelbar ist.

Die Anlagefläche 34 des Kontaktelements 30 bildet eine z-Referenz, welche eine hochpräzise Positionierung des Strahlfokus 18 in dem zu bearbeitenden Objekt ermöglicht. Zur Überprüfung der z-Kalibrierung der Laserbearbeitungseinrichtung 10 ist die Steuereinheit 22 dazu eingerichtet, die Durchführung eines Kalibrierungstests zu bewirken, bei welchem bestimmte Testbearbeitungsstrukturen mittels des Laserstrahls 14 in ein an die Anlagefläche 34 angelegtes Testobjekt 36 eingearbeitet werden, wobei die Testbearbeitungsstrukturen dauerhaft in dem Testobjekt 36 verbleiben und damit eine permanente Dokumentation des Testergebnisses ermöglichen. Nach Durchführung des Tests wird das bearbeitete Testobjekt 36 (oder zumindest ein Teil desselben) in einem schematisch bei 38 angedeuteten Archiv abgelegt. Figur 1 zeigt in dem Archiv 38 bereits einige archivierte Testobjekte, die zur besseren Unterscheidung von dem an der Anlagefläche 34 anliegenden, zu bearbeitenden Testobjekt 36 mit 36' bezeichnet sind.

Zur Erzeugung der Testbearbeitungsstrukturen enthält das Steuerprogramm (nicht näher dargestellt) der Steuereinheit 22 geeignete Instruktionen, um den Strahlfokus 18 entsprechend einem vorgegebenen Testscanmuster zu bewegen. Eine erste Ausgestaltung sieht vor, dass die Wechselwirkung der Laserstrahlung mit dem Material des Testobjekts 36 eine dauerhaft lokale Verfärbung in dem Testobjekt 36 bewirkt, so dass entsprechend dem Testscanmuster ein Verfärbungsmuster in dem Testobjekt 36 entsteht. Bei einem im sichtbaren Wellenlängenbereich und für die Laserstrahlung transparenten Testobjekt 36 kann die Verfärbung beispielsweise darin bestehen, dass das Material des Testobjekts 36 im Fokusbereich milchig wird. In einer anderen Ausgestaltung sieht das Testscanmuster die Erzeugung von Schnitten in dem Testobjekt 36 vor, insbesondere solchen Schnitten, die zu einer Zerteilung des Testobjekts 36 in mehrere Teilobjekte führen. Zur Dokumentation des Tests können dann sämtliche Teilobjekte oder nur eine Teilanzahl der Teilobjekte im Archiv 38 abgelegt werden.

Das Testobjekt 36 ist beispielsweise ein aus PMMA bestehendes Plattenstück, das durch Saugkraft an der Schnittstelleneinheit 28 gehalten werden kann. Die Schnittstelleneinheit 28 ist hierzu in nicht näher dargestellter Weise mit einem Evakuierungsanschluss ausgestattet, an den über eine Evakuierungsleitung 40 eine Evakuierungspumpe 42 anschließbar ist.

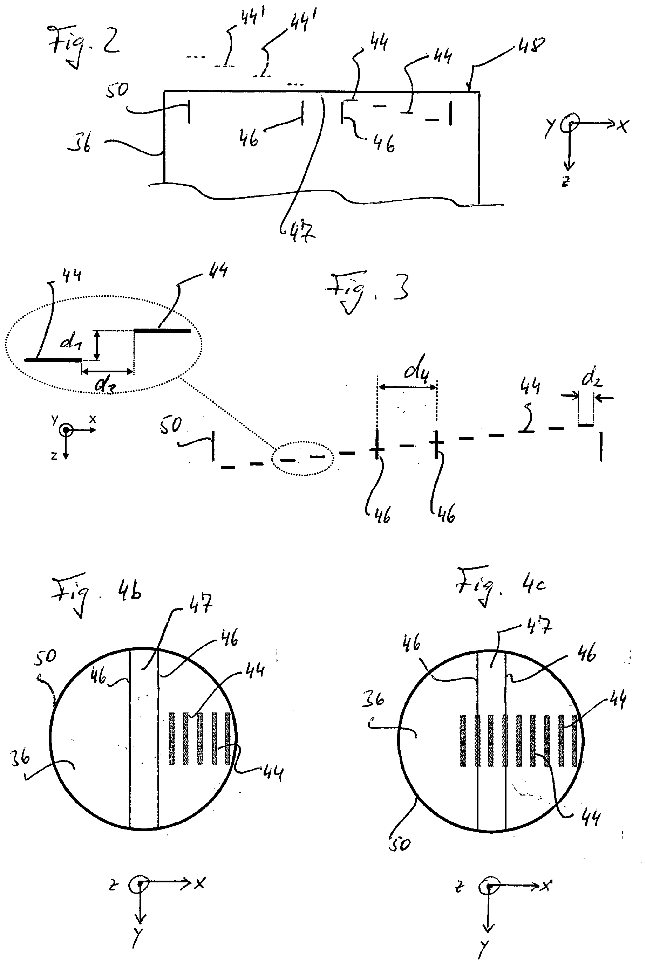

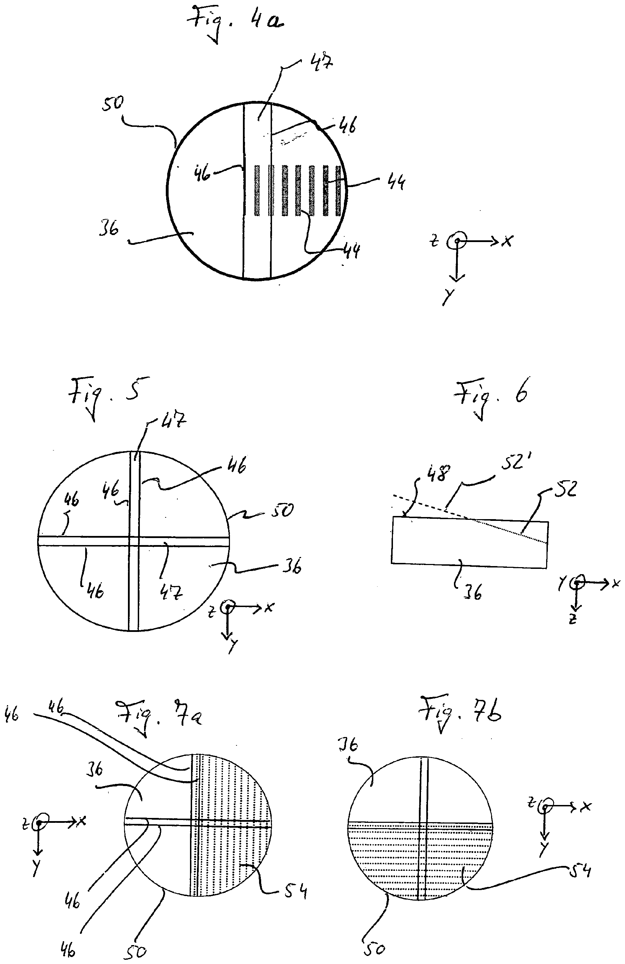

Es wird nun zusätzlich auf die Figuren 2 und 3 verwiesen. Diese Figuren stellen Verfärbungsstrukturen dar, die gemäß einem Ausführungsbeispiel mittels der Fs-Laserstrahlung der Laserbearbeitungseinrichtung 10 in das Testobjekt 36 eingebracht werden können. Die Verfärbungsstrukturen umfassen ein in z-Richtung treppenartig aufsteigendes Streifenmuster aus einer Mehrzahl jeweils parallel zur x,y-Ebene orientierter Verfärbungsstreifen 44 sowie zwei längs der x,y-Ebene parallel im Abstand nebeneinander verlaufende Referenzmarkierungen 46, die im gezeigten Beispielfall in Draufsicht auf die x,y-Ebene als Geraden erscheinen. Ungeachtet dessen können die beiden Referenzmarkierungen 46 als zweidimensional ausgedehnte Referenzstreifen- oder ebenen ausgebildet sein und eine entsprechende Erstreckung in z-Richtung besitzen. Wegen ihres in Draufsicht auf die x,y-Ebene linienförmigen Aussehens werden die beiden Referenzmarkierungen 46 nachfolgend aber als Referenzlinien bezeichnet. Diese Referenzlinien 46 definieren zwischen sich einen Soll-Durchstoßbereich 47, in welchem bei ordnungsgemäßer z-Kalibrierung der Laserbearbeitungseinrichtung 10 das von den Verfärbungsstreifen 44 gebildete Streifenmuster die der Anlagefläche 34 zugekehrte Außenfläche des Testobjekts - in Figur 2 mit 48 bezeichnet - durchstoßen sollte. Dementsprechend ist das Testscanmuster so ausgelegt, dass bei ordnungsgemäßer z-Kalibrierung der Laserbearbeitungseinrichtung 10 - bei Betrachtung in Aufstiegsrichtung des Streifenmusters - der letzte sichtbare Verfärbungsstreifen 44 in dem Soll-Durchstoßbereich 47 liegen sollte. Diese Situation ist in Figur 4a veranschaulicht. Die Figuren 4b und 4c veranschaulichen dagegen Fälle einer unkorrekten z-Kalibrierung der Laserbearbeitungseinrichtung 10, in denen das Treppenmuster der Verfärbungsstreifen 44 einmal vor dem von den Referenzlinien 46 begrenzten Soll-Durchstoßbereich 47 die Außenfläche 48 des Testobjekts 36 durchstößt (Figur 4b) und das andere Mal dahinter (Figur 4c). Aus der Steigung des Treppenmusters kann in den Fällen der Figuren 4b und 4c ohne weiteres das nötige Korrekturmaß zur Korrektur der z-Kalibrierung der Laserbearbeitungseinrichtung 10 ermittelt werden oder es kann entschieden werden, dass abgebrochen werden muss.

Beispielsweise beträgt der gegenseitige z-Abstand der Verfärbungsstreifen 44 - in Figur 3 mit d₁ bezeichnet - 5 µm. Mit einem solchen Wert für den Abstand d₁ kann erreicht werden, dass der Strahlfokus mit einer Ungenauigkeit von höchstens 5 µm im z-Richtung positioniert und kontrolliert werden kann.

Die Streifenbreite - in Figur 3 mit d₂ bezeichnet - beträgt beispielsweise etwa 250 µm. Der gegenseitige Abstand der Verfärbungsstreifen 44 in x,y-Projektion - in Figur 3 mit d₃ bezeichnet - kann beispielsweise der Streifenbreite d₂ entsprechen, im vorliegenden Fall also ebenfalls einen Wert von etwa 250 µm haben. Der gegenseitige Abstand der Referenzlinien 46 - in Figur 3 mit d₄ bezeichnet - beträgt im gezeigten Beispielfall das Vierfache der Streifenbreite d₂. Bei einer Streifenbreite von 250 µm beträgt daher das Maß d₄ 1000 µm. Über die Steigung (beispielsweise 5 µm/500 µm) kann auch eine etwa vorhandene Fehlkalibrierung berechnet werden.

Zur Erzeugung der Verfärbungsstreifen 44 in dem Testobjekt 36 definiert das Testscanmuster ein Treppenmuster, das aus einer Vielzahl den Verfärbungsstreifen 44 entsprechenden Treppenstufen besteht, wobei jede Treppenstufe von mehreren in Stufenlängsrichtung nebeneinander verlaufenden Scanlinien gebildet sein kann. Es versteht sich, dass nur diejenigen Treppenstufen dieses Treppen-Scanmusters, die innerhalb des Materials des Testobjekts 36 verlaufen, zu einem entsprechenden Verfärbungsstreifen 44 in dem Testobjekt 36 führen. Im Regelfall wird eine Teilanzahl der Treppenstufen des Treppen-Scanmusters außerhalb des Testobjekts 36 bleiben, selbst bei einer geringfügigen Fehlkalibrierung der Laserbearbeitungseinrichtung 10 in z-Richtung. Solche außerhalb des Testobjekts 36 liegenden Treppenstufen des Treppen-Scanmusters sind in Figur 2 gestrichelt bei 44' angedeutet.

Die in dem Testobjekt 36 erzeugten Verfärbungsstrukturen umfassen zudem eine im wesentlichen parallel zur x,y-Ebene liegende Kreislinie 50, welche beispielsweise einem maximal verfügbaren x,y-Scanbereich der Laserbearbeitungseinrichtung 10 entsprechen kann oder innerhalb eines solchen maximalen x, y-Scanbereichs im Abstand von dessen Außengrenzen verlaufen kann. Beispielsweise kann die Kreislinie 50 mit einem Durchmesser erzeugt werden, wie er typisch ist für einen im Rahmen einer Fs-LASIK-Behandlung lasertechnisch erzeugten kornealen Flap. Übliche Flapdurchmesser liegen beispielsweise im Bereich zwischen 9 und 11 mm. Die Kreislinie 50 kann dementsprechend beispielsweise einen Durchmesser von 10 oder 11 mm haben. Ist sie vollständig und unverzerrt in dem Testobjekt 36 als Ringverfärbung erkennbar, ist dies ein Hinweis darauf, dass zumindest der für die Flaperzeugung benötigte Scanbereich uneingeschränkt verfügbar ist.

Figur 5 zeigt eine Variante, bei der das in das Testobjekt 36 eingeschriebene Verfärbungsmuster zwei in x,y-Projektion zueinander rechtwinklige Paare von Referenzlinien 46 umfasst. Dies ermöglicht es, in zwei zueinander senkrechten Richtungen jeweils ein treppenartig oder anderweitig geradlinig ansteigendes Verfärbungsmuster in das Testobjekt 36 einzubringen.

Als Alternative zu einem treppenartig ansteigenden Verfärbungsmuster zeigt Figur 6 eine ebene Verfärbungsfläche 52, welche bei der Sichtweise der Fig. 6, die einen x,z-Schnitt darstellt, als Gerade erscheint. Die Verfärbungsfläche 52 kann beispielsweise durch eine Vielzahl in der Ebene der Verfärbungsfläche 52 nebeneinander verlaufende Linienscans des Strahlfokus realisiert werden. Diese Linienscans bilden zusammen ein ebenenhaftes Scanmuster, dessen außerhalb des Testobjekts 36 liegende Teile in Figur 6 gestrichelt bei 52' angedeutet sind. Ähnlich wie im Fall der Verfärbungsstreifen 44 kann die Kalibriergenauigkeit der Laserbearbeitungseinrichtung 10 anhand der Lage der Durchstoßlinie beurteilt werden, an welcher die Verfärbungsfläche 52 auf die Außenfläche 48 des Testobjekts 36 trifft (der Begriff des Durchstoßens ist hier bildhaft zu verstehen, da sich das von den Verfärbungsstreifen 44 gebildete Stufenmuster sowie die Verfärbungsfläche 52 natürlich nicht außerhalb des Testobjekts 36 fortsetzen; für einen auf die Außenfläche 48 schauenden Betrachter sieht es jedoch so aus, als werde die Außenfläche 48 von dem Streifenmuster bzw. von der Verfärbungsfläche 52 durchstoßen).

Die Figuren 7a und 7b veranschaulichen den Fall, dass bei einem Testobjekt mit zwei zueinander senkrechten Paaren von Referenzlinien 46 (entsprechend der Variante der Figur 5) in Zuordnung zu jedem Referenzlinienpaar eine ebene Verfärbungsfläche ähnlich der Darstellung der Figur 6 in das Testobjekt 36 eingebracht wird. Man erkennt die zur Realisierung der Verfärbungsfläche von dem Strahlfokus abgefahrenen Scanlinien. Diese sind mit 54 bezeichnet und liegen hinreichend eng beieinander, so dass sich beim Betrachter ein flächenhafter Verfärbungseindruck einstellt. Sowohl in Figur 7a als auch in Figur 7b durchstößt die jeweilige Verfärbungsfläche die Außenfläche des Testobjekts 36 innerhalb des zwischen dem betreffenden Referenzlinienpaar festgelegten Soll-Durchstoßbereichs, d.h. es liegt eine ordnungsgemäße Kalibrierung vor.

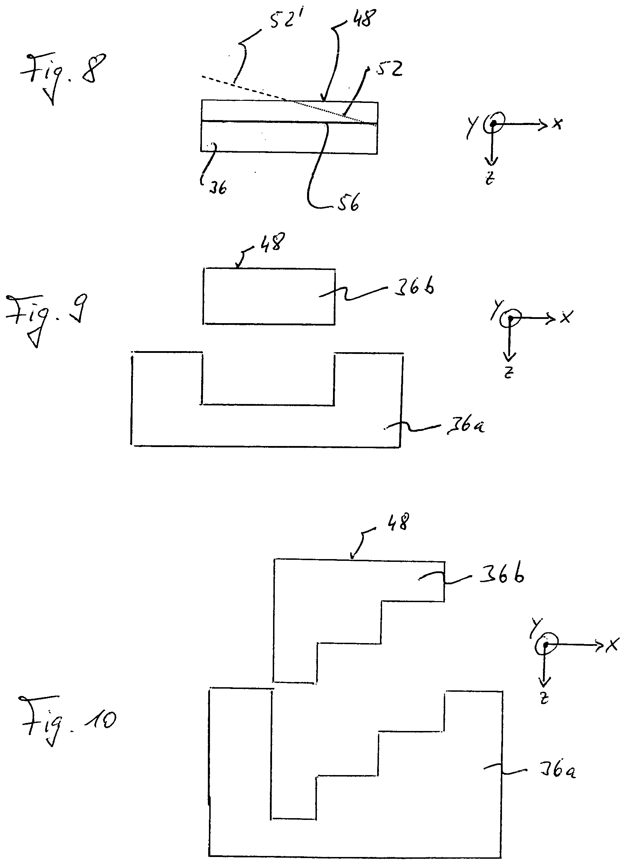

Bei der in Figur 8 gezeigten Ausführungsform ist das Testobjekt 36 mehrschichtig aufgebaut und weist eine Zwischenschicht 56 aus einem anderen Material als die in z-Richtung oberhalb und unterhalb der Zwischenschicht 56 liegenden Bereiche des Testobjekts 36 auf. Das Material der Zwischenschicht 56 zeigt eine andere Wechselwirkungsreaktion mit der im Rahmen des Kalibrierungstests eingestrahlten Laserstrahlung als die übrigen Materialbereiche des Testobjekts 36. Beispielsweise führt die Wechselwirkung der Laserstrahlung mit dem Material der Zwischenschicht 56 zu einer anderen Verfärbung als in den übrigen Materialbereichen des Testobjekts 36. Bei gegebenem z-Abstand der Zwischenschicht 56 von der Außenfläche 48 des Testobjekts 36 kann mit Hilfe des gegenseitigen Abstands der Durchstoßstellen der Verfärbungsfläche 52 durch die Außenfläche 48 und die Zwischenschicht 56 nicht nur die z-Kalibrierung auf einen etwaigen z-Offset überprüft werden, sondern auch eine Überprüfung auf richtige Skalierung der z-Achse des von der Laserbearbeitungseinrichtung 10 verwendeten Koordinatensystems vorgenommen werden. Zur eindeutigen Feststellung der Durchstoßstelle der Verfärbungsfläche 52 durch die Zwischenschicht 56 kann es vorteilhaft sein, das Testobjekt 36 mit einer polierten, vorzugsweise gradlinigen Seitenfläche auszuführen, so dass sowohl die Verfärbungsfläche 52 als auch die Zwischenschicht 56 in einer seitlichen Betrachtung erkennbar sind. Das Testobjekt 36 kann hierzu beispielsweise als Halbscheibe oder Viertelscheibe ausgeführt sein.

Die Figuren 9 und 10 zeigen in nicht maßstabsgetreuer Weise zwei Ausführungsbeispiele, die kein Teil der Erfindung darstellen, bei denen die in das Testobjekt 36 eingebrachten Bearbeitungsstrukturen Schnitte darstellen, die zu einer Zerlegung des Testobjekts 36 in Teilobjekte 36a, 36b führen. Beispielsweise kann aus der Außenfläche 48 des Testobjekts ein plattenartiges Teilobjekt (Figur 9) oder ein treppenartig gestuftes Teilobjekt (Figur 10) herausgeschnitten werden. Durch eine Dickenvermessung des herausgeschnittenen Teilobjekts 36b in z-Richtung können anschließend Rückschlüsse über die Genauigkeit der z-Kalibrierung der Laserbearbeitungseinrichtung 10 gewonnen werden. Die Dickenmessung kann beispielsweise mit optischen, akustischen oder mechanischen Messmitteln durchgeführt werden. Als Alternative zu einer Vermessung des Teilobjekts 36b ist es vorstellbar, die z-Tiefe der in dem verbleibenden Teilobjekt 36a in Folge der Herauslösung des Teilobjekts 36b entstandenen Oberflächenvertiefung zu vermessen.

Nach Anbringung der Bearbeitungsstrukturen in dem Testobjekt 36 kann die Steuereinheit 22 der Laserbearbeitungseinrichtung 10 die Ausgabe einer Aufforderung auf dem Monitor 24 bewirken, welche den Anwender auffordert, über das Eingabegerät 26 einzugeben, ob der Kalibrierungstest erfolgreich war oder nicht. Je nach Eingabe des Anwenders bewirkt die Steuereinheit 22 dann eine Freigabe oder Sperrung der Laserbearbeitungseinrichtung 10 für spätere Augenoperationen.

Bei einer bevorzugten Ausführungsform können geeignete Vorkehrungen vorgesehen sein, um Dampf oder/und Partikel, die bei der Erzeugung der Bearbeitungsstrukturen in dem Testobjekt entstehen können, abzusaugen.

## Patentansprüche

1. Verfahren zum Prüfen der Genauigkeit der Fokuspositionierung einer zur Abgabe gepulster fokussierter Laserstrahlung eingerichteten Lasereinrichtung (10), deren Fokusposition (18) sowohl in als auch quer zur Ausbreitungsrichtung der Laserstrahlung verstellbar ist, wobei die Lasereinrichtung ein für die Laserstrahlung transparentes Kontaktelement (30) mit einer Anlagefläche (34) zur Anlage eines zu bearbeitenden Objekts aufweist, wobei das Verfahren die Schritte umfasst:
- Anlegen eines zumindest in einem Bearbeitungsbereich für die Laserstrahlung transparenten Testobjekts (36) an die Anlagefläche,
- Einstrahlen der Laserstrahlung in das an der Anlagefläche anliegende Testobjekt und dabei Bewegen der Fokusposition nach Maßgabe eines vorgegebenen Testmusters zur Erzeugung dauerhafter Bearbeitungsstrukturen (44, 46, 50, 52) in dem Testobjekt,
**dadurch gekennzeichnet, dass** die Bearbeitungsstrukturen mindestens eine zu umliegenden Materialbereichen des Testobjekts (36) optisch kontrastierende erste Verfärbungsstruktur (44, 52) umfassen, welche in dem Testobjekt (36) längs einer quer, insbesondere geradlinig schräg, zur Ausbreitungsrichtung der Laserstrahlung verlaufenden Strukturerstreckungsrichtung bis zu einer der Anlagefläche (34) zugekehrten Außenfläche (48) des Testobjekts aufsteigt, dass die Bearbeitungsstrukturen ferner eine zu umliegenden Materialbereichen des Testobjekts (36) optisch kontrastierende zweite Verfärbungsstruktur umfassen, welche eine oder mehrere Referenzmarkierungen (46) zur Markierung eines Soll-Durchstoßbereichs (47) der ersten Verfärbungsstruktur durch die Außenfläche (48) des Testobjekts (36) bildet, und
dass geprüft wird, ob die erste Verfärbungsstruktur (44, 52) die Außenfläche (48) des Testobjekts (36) innerhalb oder außerhalb des Soll-Durchstoßbereiches (47) durchstößt.

2. Verfahren nach Anspruch 1, wobei die erste Verfärbungsstruktur (44) ein Streifenmuster aus einer Vielzahl längs der Strukturerstreckungsrichtung aufeinanderfolgender Verfärbungsstreifen repräsentiert.

3. Verfahren nach Anspruch 2, wobei die Verfärbungsstreifen (44) mit ihrer Streifenebene orthogonal zur Strahlungsausbreitungsrichtung orientiert sind.

4. Verfahren nach Anspruch 3, wobei paarweise aufeinanderfolgende Streifen (44) einen gegenseitigen Abstand in Strahlungsausbreitungsrichtung von höchstens 10 µm, besser höchstens 8 µm, noch besser höchstens 6 µm, beispielsweise 5 µm haben.

5. Verfahren nach einem der Ansprüche 2 bis 4, wobei paarweise aufeinanderfolgende Streifen (44) bei Projektion auf eine zur Strahlungsausbreitungsrichtung orthogonale Ebene gegenseitigen Abstand haben.

6. Verfahren nach Anspruch 1, wobei die erste Verfärbungsstruktur (52) eine schräg zur Ausbreitungsrichtung der Laserstrahlung bis zur Außenfläche des Testobjekts aufsteigende ebene Verfärbungsfläche repräsentiert.

7. Verfahren nach einem der vorhergehenden Ansprüche, wobei die Referenzmarkierungen (46) ein paar parallel im Abstand nebeneinander verlaufender Markierungslinien zur Markierung des Soll-Durchstoßbereichs (47) bilden.

8. Verfahren nach einem der vorhergehenden Ansprüche, wobei das Testmuster die Erzeugung einer zu umliegenden Materialbereichen des Testobjekts (36) optisch kontrastierenden dritten Verfärbungsstruktur (50) vorsieht, welche an der Außengrenze eines vorgegebenen verfügbaren, zur Strahlungsausbreitungsrichtung orthogonalen Positionierfelds für die Fokusposition verläuft.

9. Verfahren nach einem der vorhergehenden Ansprüche, wobei das Testobjekt (36) zumindest in seinem Bearbeitungsbereich aus einem im sichtbaren Wellenlängenbereich transparenten Material gefertigt ist.

10. Verfahren nach einem der vorhergehenden Ansprüche, wobei das Testobjekt zumindest in seinem Bearbeitungsbereich mehrschichtig aufgebaut ist und die Wechselwirkungsreaktion auf die eingestrahlte Laserstrahlung in unterschiedlichen Materialschichten des Testobjekts unterschiedlich ist.

11. Verfahren nach einem der vorhergehenden Ansprüche, ferner umfassend:
- Freigeben der Lasereinrichtung (10) für eine Laserbehandlung des humanen Auges, falls ein Erfolg der Prüfung der Lasereinrichtung festgestellt wird,
- Sperren der Lasereinrichtung für eine Laserbehandlung des humanen Auges, falls ein Misserfolg der Prüfung der Lasereinrichtung festgestellt wird,
- Feststellen des Erfolgs oder Misserfolgs der Prüfung auf Grundlage einer Benutzereingabe über ein Eingabegerät (26) der Lasereinrichtung.

## Claims

1. Method for checking the accuracy of the focus positioning of a laser device (10) configured for emitting pulsed focused laser radiation, with the focus position (18) thereof being adjustable both in the propagation direction of the laser radiation and also transversely to said propagation direction, wherein the laser device has a contact element (30) which is transparent to the laser radiation and has a placement surface (34) for placement of an object to be processed, wherein the method comprises the steps of:
- placing a test object (36) that is transparent to the laser radiation at least in one processing region against the placement surface,
- radiating the laser radiation into the test object lying against the placement surface and, in the process, moving the focus position in accordance with a specified test pattern to produce permanent processing structures (44, 46, 50, 52) in the test object,
**characterized in that** the processing structures comprise at least one first colouration structure (44, 52) that contrasts visually with surrounding material regions of the test object (36) and that rises in the test object (36) along a structure extent direction, which extends transversely, in particular rectilinearly at an angle, to the propagation direction of the laser radiation, up to an outer surface (48) of the test object facing the placement surface (34), **in that** the processing structures furthermore comprise a second colouration structure that contrasts visually with surrounding material regions of the test object (36) and forms one or more reference markings (46) for marking a target penetration region (47) of the first colouration structure through the outer surface (48) of the test object (36), and
**in that** a check is performed as to whether the first colouration structure (44, 52) penetrates the outer surface (48) of the test object (36) inside or outside of the target penetration region (47).

2. Method according to Claim 1, wherein the first colouration structure (44) represents a stripe pattern of a multiplicity of colouration stripes that follow one another along the structure extent direction.

3. Method according to Claim 2, wherein the colouration stripes (44) are oriented with their stripe plane orthogonally to the radiation propagation direction.

4. Method according to Claim 3, wherein stripes (44) that follow one another in pairs have a mutual distance in the radiation propagation direction of at most 10 pm, better yet at most 8 pm, better still at most 6 pm, for example 5 µm.

5. Method according to any of Claims 2 to 4, wherein stripes (44) that follow one another in pairs have a mutual distance upon projection onto a plane that is orthogonal to the radiation propagation direction.

6. Method according to Claim 1, wherein the first colouration structure (52) represents a planar colouration surface that rises at an angle to the propagation direction of the laser radiation, up to the outer surface of the test object.

7. Method according to any of the preceding claims, wherein the reference markings (46) form a few marking lines, running parallel next to one another at a distance, for marking the target penetration region (47).

8. Method according to any of the preceding claims, wherein the test pattern envisages the production of a third colouration structure (50) that visually contrasts with surrounding material regions of the test object (36) and extends at the outer border of a specified available positioning field for the focus position that is orthogonal to the radiation propagation direction.

9. Method according to any of the preceding claims, wherein the test object (36) is manufactured, at least in its processing region, from a material that is transparent in the visible wavelength range.

10. Method according to any of the preceding claims, wherein the test object is constructed in multiple layers at least in its processing region and the interaction reaction to the incoming laser radiation is different in different material layers of the test object.

11. Method according to any of the preceding claims, furthermore comprising:
- enabling the laser device (10) for laser treatment of the human eye if a success of the check of the laser device is established,
- disabling the laser device for laser treatment of the human eye if failure of the check of the laser device is established,
- establishing the success or failure of the check on the basis of a user input via an input device (26) of the laser device.

## Revendications

1. Procédé de contrôle de la précision du positionnement de focalisation d'un dispositif laser (10) qui est conçu pour émettre un rayonnement laser focalisé pulsé et dont la position de focalisation (18) peut être ajustée aussi bien dans la direction de propagation du rayonnement laser que transversalement à celle-ci, le dispositif laser comprenant un élément de contact (30) qui est transparent au rayonnement laser et qui comporte une surface d'appui (34) destinée à venir en contact avec un objet à traiter, le procédé comprenant les étapes suivantes :
- placer un objet à contrôler (36), qui est transparent pour le rayonnement laser au moins dans une zone de traitement, sur la surface d'appui,
- injecter le rayonnement laser dans l'objet à contrôler en appui sur la surface de contact et déplacer ainsi la position de focalisation selon un motif de contrôle spécifié pour générer des structures de traitement permanentes (44, 46, 50, 52) dans l'objet à contrôler,
**caractérisé en ce que** les structures de traitement comprennent au moins une première structure de décoloration (44, 52) qui contraste optiquement avec des zones de matériau environnantes de l'objet à contrôler (36), qui s'étend dans l'objet à contrôler (36) suivant une direction d'extension de structure qui s'étend transversalement, notamment de manière oblique et rectiligne, à la direction de propagation du rayonnement laser jusqu'à une surface extérieure (48) de l'objet à contrôler dirigée vers la surface d'appui (34), **en ce que** les structures de traitement comprennent également une deuxième structure de décoloration qui contraste optiquement avec les zones de matériau environnantes de l'objet à contrôler (36) et qui forme un ou plusieurs marquages de référence (46) destinés à marquer une zone de perforation cible (47) de la première structure de décoloration à travers la surface extérieure (48) de l'objet à contrôler (36), et **en ce qu'**un contrôle est effectué si la première structure de décoloration (44, 52) perfore la surface extérieure (48) de l'objet à contrôler (36) à l'intérieur ou à l'extérieur de la zone de perforation cible (47).

2. Procédé selon la revendication 1, la première structure de décoloration (44) représentant un motif de bandes composé d'un grand nombre de bandes de décoloration qui se succèdent suivant la direction d'extension de structure.

3. Procédé selon la revendication 2, les bandes de décoloration (44) étant orientées de sorte que leur plan de bande soit orthogonal à la direction de propagation du rayonnement.

4. Procédé selon la revendication 3, des paires de bandes successives (44) présentant un espacement mutuel dans la direction de propagation de rayonnement d'au plus 10 pm, de préférence d'au plus 8 pm, plus préférablement d'au plus 6 pm, par exemple de 5 µm.

5. Procédé selon l'une des revendications 2 à 4, des bandes successives (44) étant mutuellement espacées par paires lorsqu'elles sont projetées sur un plan orthogonal à la direction de propagation de rayonnement.

6. Procédé selon la revendication 1, **caractérisé en ce que** la première structure de décoloration (52) représente une surface de décoloration plane qui s'élève obliquement par rapport à la direction de propagation du rayonnement laser jusqu'à la surface extérieure de l'objet à contrôler.

7. Procédé selon l'une des revendications précédentes, les marquages de référence (46) formant une paire de lignes de marquage s'étendant parallèlement à distance l'une de l'autre et destinées à marquer la zone de perforation cible (47).

8. Procédé selon l'une des revendications précédentes, le motif de contrôle prévoyant la génération d'une troisième structure de décoloration (50) qui contraste optiquement avec les zones de matériau environnantes de l'objet à contrôler (36) et qui s'étend à la limite extérieure d'une zone de positionnement disponible spécifiée orthogonale à la direction de propagation de rayonnement et destinée à la position de focalisation.

9. Procédé selon l'une des revendications précédentes, l'objet à contrôler (36) étant réalisé en un matériau transparent dans le domaine des longueurs d'onde visibles au moins dans sa zone de traitement.

10. Procédé selon l'une des revendications précédentes, l'objet à contrôler ayant une structure multicouche au moins dans sa zone de traitement et la réaction d'interaction au rayonnement laser injecté étant différente dans différentes couches de matériau de l'objet à contrôler.

11. Procédé selon l'une des revendications précédentes, comprenant en outre les étapes suivantes :
- débloquer le dispositif laser (10) pour un traitement laser de l'œil humain si le contrôle du dispositif laser est effectué avec succès,
- bloquer le dispositif laser pour un traitement laser de l'œil humain si le contrôle du dispositif laser est un échec,
- déterminer si le contrôle est un succès ou un échec sur la base d'une entrée utilisateur par le biais d'une unité d'entrée (26) du dispositif laser.
